Europäisches Patentamt

European Patent Office

Office européen des brevets

(19)

(11) Numéro de publication: **0 307 279**
**A2**

(12)

# DEMANDE DE BREVET EUROPEEN

(21) Numéro de dépôt: 88402175.9

(51) Int. Cl.⁴: **A 61 K 31/445**

(22) Date de dépôt: 29.08.88

(30) Priorité: 09.09.87 FR 8712486

(43) Date de publication de la demande:
15.03.89 Bulletin 89/11

(84) Etats contractants désignés:
AT BE CH DE FR GB IT LI LU NL SE

(71) Demandeur: FOURNIER INNOVATION ET SYNERGIE
38, avenue Hoche
F-75008 Paris (FR)

(72) Inventeur: Pascal, Marc
résidant 32 Cours du Park
F-21000 Dijon (FR)

(74) Mandataire: Clisci, Serge et al
S.A. FEDIT-LORIOT CONSEILS EN PROPRIETE
INDUSTRIELLE 38, avenue Hoche
F-75008 Paris (FR)

(54) **Nouvelle utilisation de la (5-amino-2-(4-méthyl-1-pipéridinyl)phényl)-(4-chlorophényl)méthanone en tant qu'agent antiviral.**

(57) La présente invention concerne l'utilisation de la (5-amino-2-(4-méthyl-1-pipéridinyl)phényl)-(4-chlorophényl)-méthanone en tant qu'inhibiteur de l'infectivité virale.

EP 0 307 279 A2

Bundesdruckerei Berlin

**Description**

## NOUVELLE UTILISATION DE LA (5-AMINO-2-(4-METHYL-1-PIPERIDINYL)PHENYL)-(4-CHLOROPHENYL)-METHANONE EN TANT QU'AGENT ANTIVIRAL

La présente invention concerne une nouvelle utilisation de la (5-amino-2-(4-méthyl-1-pipéridinyl)-phényl)-(4-chloro-phényl)-méthanone en tant qu'inhibiteur de l'infectivité virale.

On connaît par EP-69 012 la (5-amino-2-(4-méthyl-1-pipéridinyl)phényl)-(4-chlorophényl)-méthanone en tant qu'agent immunostimulant et immunoadjuvant, et qu'on désignera dans ce qui suit comme étant le produit.

De façon surprenante, on vient à présent de découvrir que ledit produit possède des propriétés virucides qui se manifestent par une inhibition de l'infectivité virale.

On a en particulier trouvé selon l'invention que ledit produit possède la propriété d'inhiber l'infectivité de cellules mononuclées périphériques humaines par le rétrovirus "Human Immunodeficiency Virus" (en abrégé HIV) appartenant à la famille des lentivirus.

D'une manière générale ledit produit est efficace vis-à-vis des virus proprement dits et des rétrovirus notamment ceux appartenant à la famille des lentivirus et ceux appartenant à celle des oncovirus.

Il agit en particulier sur le virus de l'herpès (notamment le HSV-II), le virus de l'hépatite B (en abrégé HBV), les virus du SIDA (notament les HIV 1 et HIV 2), les virus de leucémies humaines à lymphocyte T (en abrégé HTLV, notamment les HTLV-I et HTLV-II), le virus de la leucémie féline (en abrégé FeLV), le virus de l'anémie infectieuse équine, le virus de Epstein-Barr, le virus de l'arthrite caprine et le virus VISNA du mouton.

On trouvera, ci-après, les protocoles expérimentaux qui ont permis de mettre en évidence la nouvelle propriété de la (5-amino-2-(4-méthyl-1-pipéridinyl)phényl)-(4-chlorophényl)-méthanone.

Pour chacun des trois protocoles qui suivent, on a préparé une solution mère de (5-amino-2-(4-méthyl-1-pipéridinyl)phényl)-(4-chlorophényl)-méthanone à 100 mg/l dans de l'eau physiologique contenant 9 g/l de chlorure de sodium et acidifiée à pH = 3 par de l'acide chlorhydrique. La solution mère est ensuite diluée dans un milieu RPMI 1640 jusqu'à la concentration désirée. La solution ainsi obtenue est stérilisée par filtration sur membrane "MILLIPORE" ayant un diamètre moyen de pores de 0,22 m.

A partir de cellules T lymphoblastoïdes (lignée CEM) infectées par un virus HIV et donc susceptibles de produire ledit virus HIV, on recueille ce dernier par des moyens appropriés à partir d'un surnageant de culture desdites cellules.

Les cellules utilisées sont des lymphocytes de sujets normaux HIV négatifs isolés à partir du sang périphérique par gradient de Ficoll puis stimulés pendant trois jours par de la phytohémagglutinine p (en abrégé pHAp) avant leur utilisation.

Les lymphocytes sont ensuite cultivés dans le milieu RPMI 1640 contenant 10 % de sérum de veau foetal, 10 % d'interleukine II, 2 g/ml de polybrène, 1 % de glutamine, 1 % d'antibiotiques et une dilution au 1/2500 de sérum anti-interféron humain.

La production du virus HIV par des cellules T humaines traitées ou non par le produit a été régulièrement suivie par mesure de l'activité transcriptase inverse, représentative de la réplication virale, et présente dans 1 ml de surnageant de culture. On concentre 100 fois 1 ml de surnageant par ultracentrifugation puis on détermine l'activité enzymatique de cet échantillon dans 50 l d'un mélange réactionnel contenant 50 mM de tampon tris (hydroxyméthylaminométhane) de pH = 7,9, 20 mM de KCl, 5 mM de $MgCl_2$, 1 mM de 1,4-dithiothréitol, 0,05 unité de densité optique par ml d'acide polyadénylique (5'), 0,05 unité de densité optique par ml d'acide oligodéoxythymidylique 12-18, 5 Ci de $^3$HTTP et 0,1 % de Triton X 100. Après une heure d'incubation à 37°C, les produits acido-insolubles sont précipités par de l'acide trichloracétique à 20 %, filtrés sur "MILLIPORE" 0,45 m, puis la radioactivité contenue dans ces produits de réaction est mesurée à l'aide d'un compteur à scintillation Kontron.

Trois protocoles ou types de traitement sont effectués simultanément, chacun desdits traitements étant effectué avec une dose prédéterminée de produit (5-amino-2-(4-méthyl-1-pipéridinyl)phényl)-(4-chlorophényl)-méthanone.

Un témoin sans produit est également effectué de la même manière que pour les trois protocoles qui sont donnés ci-après :

**I - PROTOCOLE 1**

Il s'agit d'un pré-traitement avant l'infection virale.

On prépare trois suspensions de 3 ml chacune de lymphocytes ($10^6$ cellules/ml) préalablement stimulés par de la pHAp. Une suspension est traitée par une dose de 0,1 g/ml, l'autre par 0,05 g/ml et la dernière par 0,01 g/ml de produit pendant 18 heures. Les cellules de chaque culture ainsi obtenue sont ensuite lavées pour éliminer le produit avant d'être infectées par du virus HIV 1 dont l'activité transcriptase inverse est de 5000 cpm/$10^6$ cellules.

Après une heure d'adsorption virale, les trois suspensions infectées et le témoin sont ajustés à $10^6$ cellules/ml dans le milieu de culture.

On cultive les cellules des trois suspensions et du témoin pendant trente jours à raison d'un passage (ou repiquage) tous les trois ou quatre jours. A chaque passage, la production de virus HIV 1 est suivie par mesure de l'activité transcriptase inverse présente dans 1 ml de surnageant de chaque culture et comparée à celle observée pour la culture témoin.

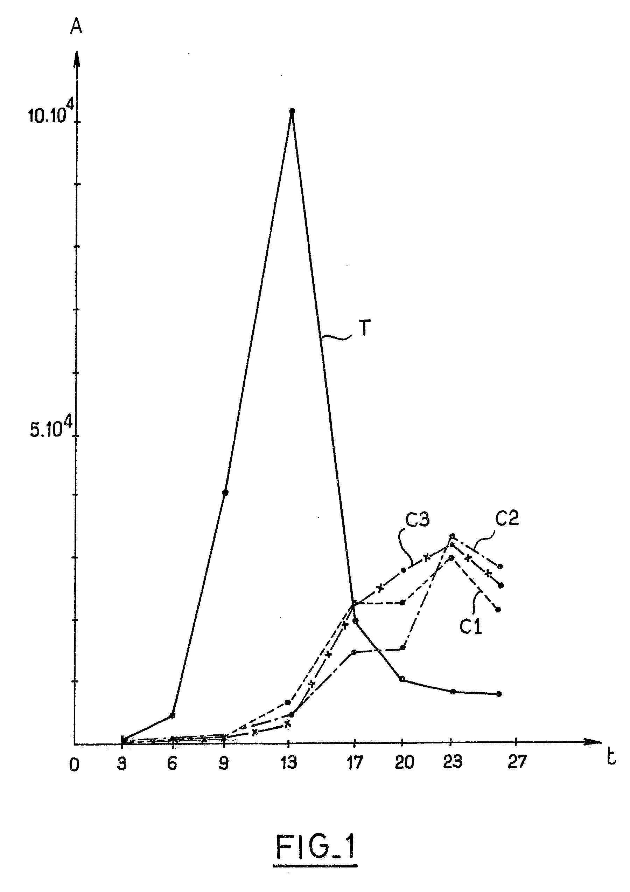

Les figures 1 à 3 qui représentent l'activité transcriptase inverse (A exprimée en cpm/ml) en fonction du temps (t exprimé en nombre de jours), comprennent chacune quatre courbes :
T représentant l'activité transcriptase inverse dans la culture témoin.
$C_1$ représentant l'activité transcriptase inverse pour la culture traitée par une dose de 0,1 g/ml de produit.
$C_2$ représentant l'activité transcriptase inverse pour la culture traitée par une dose de 0,05 g/ml de produit, et
$C_3$ représentant l'activité transcriptase inverse pour la culture traitée par une dose de 0,01 g/ml de produit.

La figure 1 montre qu'un pré-traitement de lymphocytes humains par des doses croissantes de produit provoque une inhibition partielle du pouvoir infectieux du virus HIV 1 pour lesdits lymphocytes quelle que soit la dose. En effet, il suffit de déterminer le rapport de la valeur de l'activité transcriptase inverse de la culture témoin (courbe T) à chacune des valeurs des activités transcriptase inverse des autres cultures, pour constater que déjà une assez sensible inhibition de l'infectivité virale a été obtenue.

## II - PROTOCOLE 2

Il s'agit d'un traitement pendant l'adsorption virale.

Trois suspensions de 3 ml chacune de lymphocytes humains préalablement stimulés par de la pHAp sont infectées par du virus HIV 1 dont l'activité transcriptase inverse est de 5 000 cpm/$10^6$ cellules en même temps qu'est introduit le produit à des doses de 0,1, 0,05 ou 0,01 g/ml respectivement.

Après une heure à 37°C, les lymphocytes sont lâvés puis remis en culture à raison de $10^6$ cellules/ml. La production virale est suivie de façon identique au protocole 1 précédent.

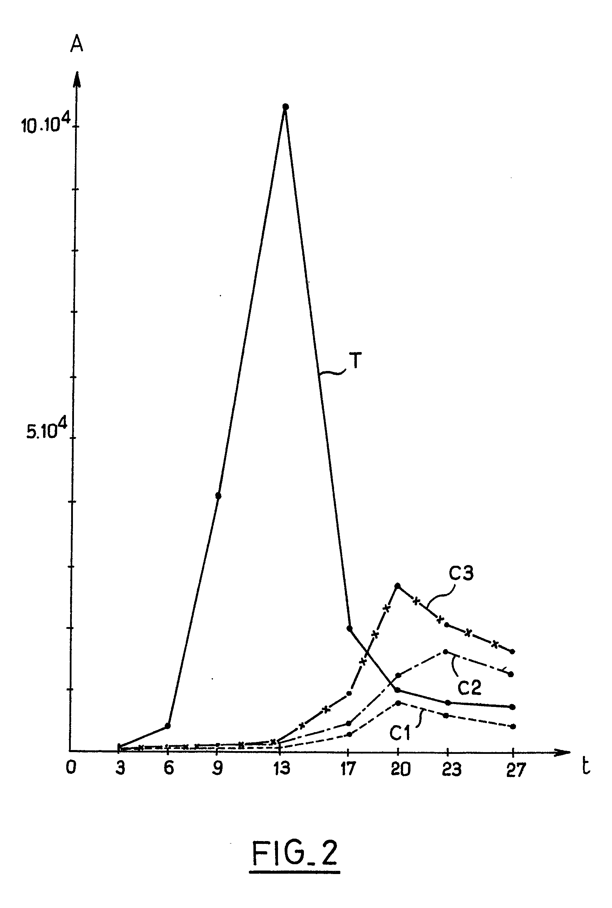

La figure 2 est représentative de l'activité transcriptase inverse en fonction du temps donné en nombre de jours et relative au protocole 2.

La figure 2 montre, lorsque l'adsorption du virus HIV 1 sur des lymphocytes humains est effectuée en présence de diverses concentrations du produit (courbes $C_1$ à $C_3$), une inhibition très significative du pouvoir infectieux du virus HIV 1 pour les cellules T humaines.

Il est aisé de constater encore que les rapports des valeurs des activités transcriptase inverse déterminés comme précédemment indiqué à propos de la figure 1, sont relativement importants.

L'inhibition constatée semble être proportionnelle à la dose utilisée et elle est maximale à la concentration la plus forte étudiée (0,1 g/ml, courbe $C_1$ de la figure 2). Pour les autres doses (0,05 g/ml - courbe $C_2$, et 0,01 g/ml - courbe $C_3$), l'inhibition est partielle, comme l'indiquent le retard dans l'apparition du pic de production virale et la diminution de cette production virale.

## III - PROTOCOLE 3

Il s'agit d'un traitement après adsorption virale (post-traitement).

Trois suspensions de 3 ml chacune de lymphocytes humains préablement stimulés par de la pHAp sont infectées par du virus HIV 1 dont l'activité transcriptase inverse est de 5 000 cpm/$10^6$ cellules. Après une heure d'adsorption virale à 37°C, les lymphocytes sont remis en culture à raison de $10^6$ cellules/ml dans du milieu de culture contenant 0,1 0,05 ou 0,01 g/ml de produit respectivement. Trois jours après et sept jours après l'infection virale, les cellules sont centrifugées puis remises en culture à raison de $10^6$ cellules/ml dans du milieu de culture contenant du produit aux doses précitées. A partir du dixième jour après l'infection virale, les passages cellulaires sont effectués tous les trois ou quatre jours sans addition de produit. La production virale est suivie de façon identique aux protocoles précédents.

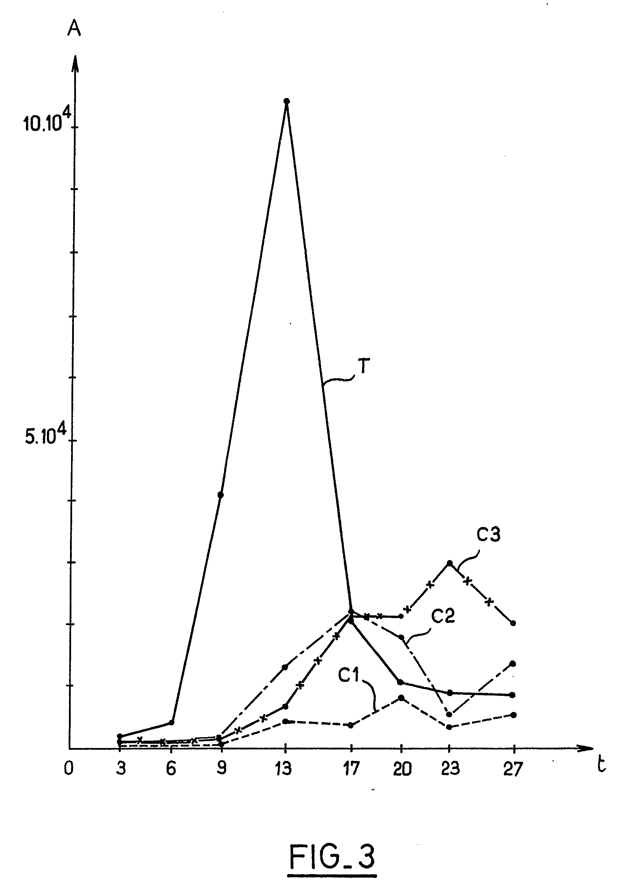

- La figure 3 est représentative de l'activité transcriptase inverse relative au protocole 3.

Lorsque les lymphocytes humains sont traités pendant trois passages après l'adsorption virale par des doses croissantes de produit (courbe $C_1$ pour la dose de 0,1 g/ml, courbe $C_2$ pour la dose de 0,05 g/ml et courbe $C_3$ pour la dose de 0,01 g/ml), une inhibition de l'infection virale et vraisemblablement une inhibition de la multiplication du HIV 1 dans ces lymphocytes est observée. Elle est réellement très significative lors d'un traitement à 0,1 g/ml, car le rapport des pics des courbes T et $C_1$ est relativement élevé, de l'ordre de 10. Elle est partielle pour les autres doses.

Lors du pré-traitement par le produit, quelle que soit la dose, on observe un retard important dans l'apparition du pic de transcriptase inverse et une inhibition partielle du pouvoir infectieux du virus par rapport à la culture témoin n'ayant pas reçu de produit.

Dans le traitement simultané à l'adsorption virale et dans le post-traitement, on observe une inhibition très significative du pouvoir infectieux du virus, cette inhibition étant maximale à la dose la plus forte étudiée, quel que soit le temps de culture.

Ces résultats suggèrent que le produit agit sur le virus et/ou la réplication dudit virus.

Une série d'essais complémentaires a été réalisée pour mettre en évidence que le produit suivant l'invention agit sur les virus proprement dits tels que le virus de l'herpès simplex.

## EVALUATION DU PRODUIT DANS L'INFECTION SYSTEMIQUE PAR LE VIRUS DE L'HERPES SIMPLEX II (HSV-II) CHEZ LA SOURIS

Administré par voie orale (dans l'eau de boisson) aux doses quotidiennes de 4 mg/l et 20 mg/l pendant 8 jours avant l'infection par voie I.P. chez la souris de HSV-II, le produit suivant l'invention exerce un effet protecteur vis-à-vis de la mortalité induite par le HSV-II. On constate en particulier que 22 souris sur 50 et 18 souris sur 50 meurent parmi les lots recevant la dose de 4 mg/l et respectivement de 20 mg/l du produit suivant l'invention, par rapport au lot témoin ne recevant que de l'eau de boisson avant l'infection par HSV-II dans lequel 35 souris sur 50 meurent. On constate par ailleurs que, parmi les souris ayant survécu, seulement 10 souris sur 28 et 13 souris sur 32 dans les lots traités recevant la dose de 4 mg/l et respectivement 20 mg/l présentent une infection latente par comparaison aux 9 souris sur 15 du lot témoin.

Selon l'invention, on envisage l'utilisation de la (5-amino-2-(4-méthyl-1-pipéridinyl)phényl)-(4-chlorophényl)-méthanone en tant que produit pour l'obtention d'un médicament virucide destiné à une utilisation en thérapeutique humaine ou vétérinaire vis-à-vis des infections virales. L'utilisation vétérinaire concerne les animaux à sang chaud et parmi ceux-ci les mammifères.

On envisage notamment l'utilisation dudit produit pour l'obtention un médicament virucide destiné à une utilisation en thérapeutique humaine vis-à-vis de l'infectivité de cellules humaines par les rétrovirus, notamment les lentivirus, comme par exemple les virus HIV 1, HIV 2, HTLV-I et HTLV-II.

On envisage encore l'utilisation dudit produit pour l'obtention d'un médicament virucide destiné à une utilisation en thérapeutique animale vis-à-vis des infections virales, notamment vis-à-vis de l'infectivité de cellules animales par le virus FeLV et le virus VISNA de mouton.

D'une façon générale, le produit est utile en thérapeutique humaine ou animale pour le traitement des maladies dont l'origine est virale, notamment les syndrômes apparentés au SIDA, les formes asymptomatiques des infectivités par les virus HIV, le SIDA, certains cancers comme par exemple les leucémies humaines à lymphocytes T ou la leucémie féline, l'anémie infectieuse équine, les infections par le virus de Epstein-Barr, l'arthrite caprine, l'hépatite de type B, le virus de l'herpès et les infections par les virus VISNA.

La posologie quotidienne préconisée pour l'administration du produit par voie orale est de 1 à 10 mg/kg en une ou plusieurs prises.

Suivant l'invention, on préconise donc l'utilisation de la (5-amino-2-(4-méthyl-1-pipéridinyl)-phényl)-(4-chlorophényl)-méthanone pour la fabrication d'un médicament virucide dans un conditionnement accompagné d'une notice en vue de son emploi dans le traitement ou la prévention de maladies d'origine virale chez les humains ou les animaux, pour la fabrication de moyens destinés à combattre les maladies d'origine virale chez les humains ou les animaux, et dans le traitement des maladies d'origine virale chez les humains ou les animaux.

**Revendications**

1) Utilisation de la (5-amino-2-(4-méthyl-1-pipéridinyl)-phényl)-(4-chlorophényl)-méthanone pour l'obtention d'un médicament virucide destiné à une utilisation en thérapeutique humaine ou vétérinaire vis-à-vis des infections virales.

2) Utilisation selon la revendication 1, caractérisée en ce que lesdites infections virales sont des infections rétrovirales.

3) Utilisation de la (5-amino-2-(4-méthyl-1-pipéridinyl)-phényl)-(4-chlorophényl)-méthanone pour l'obtention d'un médicament virucide destiné à une utilisation en thérapeutique humaine vis-à-vis de l'infectivité de cellules humaines par les rétrovirus.

4) Utilisation selon la revendication 3 caractérisée en ce que lesdits rétrovirus sont des lentivirus.

5) Utilisation selon les revendications 3 ou 4, caractérisée en ce que lesdits rétrovirus ou lentivirus sont des virus HIV.

6) Utilisation selon les revendications 3 ou 4, caractérisée en ce que lesdits rétrovirus ou lentivirus sont des virus HTLV.

7) Utilisation de la (5-amino-2-(4-méthyl-1-pipéridinyl)-phényl)-(4-chlorophényl)-méthanone pour l'obtention d'un médicament virucide destiné à une utilisation en thérapeutique vétérinaire vis-à-vis de l'infectivité de cellules animales par les rétrovirus.

8) Utilisation selon la revendication 7, caractérisée en ce que lesdits rétrovirus sont des virus FeLV.

9) Utilisation selon la revendication 7, caractérisée en ce que lesdits rétrovirus sont des virus VISNA.

10) Utilisation de la (5-amino-2-(4-méthyl-1-pipéridinyl)-phényl)-(4-chlorophényl)-méthanone suivant la revendication 1, pour l'obtention d'un médicament antiviral destiné à une utilisation en thérapeutique humaine ou vétérinaire vis-à-vis des infections dues aux virus proprement dits.

11) Utilisation suivant la revendication 10, vis-à-vis des infections dues au virus de l'herpès.

12) Utilisation suivant la revendication 11, vis-à-vis des infections dues au virus HSV-II.

13) Utilisation suivant la revendication 1, vis-à-vis des infections dues aux virus choisi parmi l'ensemble constitué par le virus de l'herpès (notamment le HSV-II) les virus HIV (notamment les HIV 1 et HIV 2) les HTLV, (notamment les HTLV-I et HTLV-II), le virus HBV, le virus FeLV, le virus de l'anémie infectieuse équine, le virus de l'arthrite caprine, le virus d'Epstein-Barr et le virus VISNA de mouton.

FIG_1

FIG_2

FIG_3